# EUROPEAN PATENT SPECIFICATION

(11) **EP 4 611 627 B1**
(45) Date of publication and mention of the grant of the patent: **11.02.2026**
(21) Application number: 23800352.9
(22) Date of filing: 30.10.2023
(51) Int. Cl.: A61B 5/024, A61B 5/11, G16H 50/20, G16H 40/40, G16H 40/63

(54) **METHOD FOR SYNCHRONISING ACCELEROMETER-BASED METRICS USING CARDIAC ACTIVITY IN WRIST WORN WEARABLES**
VERFAHREN ZUR SYNCHRONISATION VON BESCHLEUNIGUNGSMESSERBASIERTEN METRIKEN UNTER VERWENDUNG VON HERZAKTIVITÄT IN AM HANDGELENK GETRAGENEN WEARABLES
PROCÉDÉ DE SYNCHRONISATION DE MESURES SE FONDANT SUR UN ACCÉLÉROMÈTRE UTILISANT L'ACTIVITÉ CARDIAQUE DANS DES DISPOSITIFS HABITRONIQUES PORTÉS AU POIGNET

(30) Priority: 31.10.2022 US 202263420805 P
(43) Date of publication of application: 10.09.2025
(73) Proprietor: Koninklijke Philips N.V., 5656 AG Eindhoven (NL)
(72) Inventor: GELISSEN, Jozef Hubertus, 5656 AG Eindhoven (NL); DOS SANTOS DA FONSECA, Pedro Miguel Ferreira, 5656 AG Eindhoven (NL); CERNY, Andreas, 5656 AG Eindhoven (NL); RIDGWAY, Peter Emanuel, 5656 AG Eindhoven (NL)
(74) Representative: Philips Intellectual Property & Standards
(86) International application number: PCT/EP2023/080172
(87) International publication number: WO 2024/094586

(56) References cited:
- CN-B- 109 222 990
- NL-B9- 1 043 026
- TW-U- M 625 190
- US-A1- 2021 177 286
- FLETCHER R R ET AL: "iCalm: Wearable Sensor and Network Architecture for Wirelessly Communicating and Logging Autonomic Activity", IEEE TRANSACTIONS ON INFORMATION TECHNOLOGY IN BIOMEDICINE, IEEE SERVICE CENTER, LOS ALAMITOS, CA, US, vol. 14, no. 2, 1 March 2010 (2010-03-01), pages 215 - 223, XP011327668, ISSN: 1089-7771, DOI: 10.1109/TITB.2009.2038692

## Description

### BACKGROUND

The present embodiments relate generally to accelerometer-based metrics and more particularly, to a method for synchronizing accelerometer-based metrics using cardiac activity in wrist-worn wearables.

Atopic dermatitis is a long-term type of inflammation of the skin resulting in itchy, red, swollen and cracked skin. Typically, this condition starts in childhood with changing severity over the years. Scratching the affected areas worsens the symptoms and those affected have an increased risk of skin infections. Medication to reduce itchy feeling exists, however, dermatologists may be hesitant putting patients on such medication because feedback on its efficacy by patients is vague.

It would be desirable to provide an improved method that addresses, for example, in one special-use case, the issue of monitoring patients with atopic dermatitis for the use of drug doses and providing corresponding efficacy feedback to a healthcare provider (e.g., a dermatologist).

With many Bluetooth^{™} enabled wearable devices, some drift of a respective device's clock is allowable before correcting the time of the respective device, for example, having a drift on the order of 5 minutes being acceptable. However, for some specific use cases, a drift of more than a few seconds difference between the clocks of two wearable devices is not tolerable or allowable (note that clock accuracy with respect to real-time is less relevant). Thus, it would be desirable to provide for a clock-time correction before processing data of two wearable devices.

Although implementing clock-time correction could in theory be achieved by equipping both wearable devices with some means of communication between each other, and thereby synchronize (at the start, or periodically) the data recordings, this would introduce either additional costs (i.e., to equip a generic wearable device with such means), or software complexity (i.e., to establish pairing between the two wearable devices, implement the same synchronization protocol between the two wearable devices, and perform the actual clock synchronization).

Allowing the data recordings to be synchronized a posteriori, after the data recordings are complete, would alleviate these requirements. Methods for a posteriori signal synchronization based on artefacts introduced by body movements (or based on the measurement of body movements themselves) are known; however, these are less adequate for this particular use case, since the wearable devices are worn on different extremities of the body, and the scratching behaviour is obviously different between the two hands (e.g., a user will likely not scratch with both hands simultaneously).

Accordingly, an improved method and apparatus for overcoming the problems in special-use cases in the art is desired.

Methods for synchronizing recordings of wearable devices according to the state of the art are for instance described in CN109222990B and US2021/01777286A1.

### SUMMARY

The embodiments of the present disclosure advantageously provide a method to address, for example, in one special-use case, the issue of monitoring patients with atopic dermatitis for the use of drug doses and providing corresponding efficacy feedback to a healthcare provider (e.g., a dermatologist).

For specific use cases, such as measuring scratching behaviour, patients need to wear two wearable devices to assess left- and right- hand scratching. In addition, for such specific use cases, it is important to have both wearable devices in sync with one another to assess the severity of scratching behaviour. To do this, the embodiments of the present disclosure make use of the cardiac activity simultaneously measured by both wearable devices (e.g., with an optical heart rate sensor on board each wearable device) which will independently register the same variations in cardiac activity. In other words, data which is captured collectively by both wearable devices can advantageously be synchronized, a posteriori, based on the variations in cardiac activity, as will be discussed further herein.

By monitoring an additional physiological characteristic that is, on the one hand, available at the same location where movements (or accelerations) are captured, and on the other, similar between the two, a posteriori synchronization can be guaranteed. One such characteristic is cardiac activity, which can be measured with a reflective photoplethysmography sensor, and which naturally occurs simultaneously and similarly in both hands.

According to the invention, there is provided a method for synchronizing recordings of at least two wearable devices worn by a user in a specific use case. The specific use case involves a hub executable app being executed on a hub device and designed to measure a characteristic behaviour of the user or designed to perform a surrogate measure of a transient physical characteristic over one or more periods of time. The method comprises: acquiring first recordings via a first wearable device, acquiring concurrent second recordings via a second wearable device, and syncing the first wearable device and the second wearable device, via separate syncing operations, with the hub executable app being executed on the hub device for the specific use case. The first wearable device is operable via a first clock internal to the first wearable device. The second wearable device operable via a second clock internal to the second wearable device, wherein the first clock is characterized by a first clock drift and offset that may be different from a second clock drift and offset of the second clock. The method further includes implementing a clock-time correction, specific to the characteristic behaviour being measured or surrogate measure of the transient physical characteristic, of the recordings of both wearable devices via the hub executable app before processing the respective recordings for the specific use case to obtain a measure of the characteristic behaviour or the surrogate measure of the transient physical characteristic. The clock-time correction ensures that the respective recordings have been corrected in time so that a difference between the respective clocks, or clock drift, of the two wearable devices is no more than a threshold duration of a shortest event specific to the characteristic behaviour being measured or the surrogate measure of the transient physical characteristic.

The method further includes wherein the first recordings comprise at least accelerometer and photoplethysmographic (PPG) data as a function of time per the first clock, and wherein the second recordings comprise at least accelerometer and photoplethysmographic (PPG) data as a function of time per the second clock. The method further includes wherein implementing the clock-time correction comprises aligning the first and second clocks based on a time series of interbeat intervals derived from PPG data detected via a PPG sensor of each respective wearable device. The clock-time correction based on the time series of interbeat intervals further comprises: (1) for each recording, automatically detect and represent (a) temporal locations of heart beats on each PPG signal trace and (b) interbeat intervals as two non-equally sampled time series, each sample in each time series indicating a pair that comprises (time, interbeat interval), wherein "time" corresponds to a clock time or temporal location of each detected heartbeat, and "interbeat interval" corresponds to a time distance to a next heart beat or a previous heart beat; (2) interpolate each time series to a fixed sampling rate 'fs'; (3) select one of the two PPG signal traces from the recordings of the respective wearable devices as a "master" and use the clock of the wearable device which corresponds with the selected master PPG signal trace as a master clock that from then on will be used as a reference for absolute time; and (4) synchronize the offset and clock drift between the two interpolated time series signals.

In an embodiment, synchronization of the offset and clock drift comprises: (a) finding a delay, whether positive or negative, of the "slave" signal that maximizes a correlation between the two interpolated time series signals, wherein finding the delay comprises finding a delay that gives a maximum cross-correlation between the two interpolated time series signals; (b) applying a multiplication factor to the fixed sampling rate 'fs' to compensate for whatever drift the slave clock might have; and (c) repeating steps (a) and (b) in search of the multiplication factor, within a specified time interval, to find an optimal multiplication factor and delay that gives an overall highest cross-correlation between the two interpolated time series signals.

According to another embodiment, the method further comprises: applying the optimal multiplication factor, relating to clock drift, and delay, relating to offset, to the accelerometer signal of the slave device to obtain two accurately synchronized recordings of acceleration, which can thereby be used to process the respective recordings for the specific use case to obtain the measure of the characteristic behaviour and/or detect given use case events.

In yet another embodiment, the method includes wherein implementing the clock-time correction comprises aligning the first and second clocks further based on cardiac activity that includes a heart rate derived feature based on the PPG data detected via a heart rate sensor of each respective wearable device. With this embodiment, implementing the clock-time correction further comprises: determining, as a function of a given length of the recordings, whether a threshold number of heart beats, over at least two different intervals of predetermined duration, are detectable on each of at least two different portions, to include at least one proximal portion near a beginning and one distal portion near an ending, of the recordings from each respective wearable device; and if so, then using the time series of interbeat intervals to compute the drift and clock offset; otherwise, reverting to use of the cardiac activity to compute the drift and clock offset.

In still another embodiment, the clock-time correction based on the cardiac activity further comprises: aligning a heart rate derived feature trace of the first recordings with a heart rate derived feature trace of the second recordings via shifting one trace in time with respect to the other trace by an amount such that an amplitude of the heart rate derived feature traces of the first and second recordings overlap and match within a given threshold percentage.

In a further embodiment, wherein prior to syncing, the method further comprises pairing the first wearable device and the second wearable device, via separate pairing operations, with the hub executable app being executed on the hub device, wherein the hub device comprises at least one of a smartphone, a dedicated hub device, and direct-to-cloud connectivity device. In one embodiment, the wearable devices comprise Bluetooth^{™} enabled wearable devices configured to sync with the hub executable app executed on the hub device, but not with each other.

According to another embodiment, each wearable device is equipped with means for paired wireless communication with the hub executable app executed on the hub device, but not with each other. In one embodiment, the means for paired wireless communication comprises a Bluetooth^{™} paired wireless communication module of the respective wearable device, and wherein the hub device comprises at least one of a smartphone, a dedicated hub device, and direct-to-cloud connectivity device. In another embodiment, syncing comprises transferring respective recordings of each of the first and second wearable devices from each respective wearable device to the hub executable app being executed on the hub device.

In accordance with a further embodiment, the specific use case comprises monitoring scratching behaviour and measuring movements associated with scratching via the first and second wearable devices, further wherein recordings of both wearable devices are used to calculate metrics which include one or more of total scratching duration, scratching events and hand usage. In another embodiment, the characteristic behaviour comprises scratching behaviour, the recordings comprise twenty hertz (20 Hz) data for a one-night synchronization, the duration of the shortest scratching event is two seconds (2 s), and the threshold duration is no more than one second (1 s).

In yet another embodiment, the at least two wearable devices are separate and distinct from one another, and void of an ability to be directly paired with each other. The hub executable app is configured to generate a dashboard of specific use case information based on collected and analyzed data regarding characteristics/conditions under examination over at least one predetermined duration of time. In one embodiment, the specific use case comprises scratching behaviour, and wherein the dashboard includes a weekly average of one or more of sleep time, scratching duration, percentage scratching duration to baseline, scratching events, percentage scratching events to baseline, and wear compliance at night, and percentage usage of left-hand, right-hand and both left- and right-hand for the scratching behavior.

According to an aspect of the invention, there is also provided a non-transitory computer readable medium with instructions executable by a processor for causing the processor to carry out the method according to claim 1, for synchronizing recordings of at least two wearable devices worn by the user in the specific use case designed to measure the characteristic behaviour of the user or designed to perform a surrogate measure of a transient physical characteristic over one or more periods of time.

The embodiments of the present disclosure advantageously solve the problems overcome by synchronizing accelerometer-based metrics using cardiac activity in wrist-worn wearables. Still further advantages and benefits will become apparent to those of ordinary skill in the art upon reading and understanding the following detailed description.

### BRIEF DESCRIPTION OF THE DRAWINGS

The embodiments of the present disclosure may take form in various components and arrangements of components, and in various steps and arrangements of steps. Accordingly, the drawings are for purposes of illustrating the various embodiments and are not to be construed as limiting the embodiments. In the drawing figures, like reference numerals refer to like elements. In addition, it is to be noted that the figures may not be drawn to scale.
Figure 1 is a schematic block diagram of various elements used in a method for synchronizing recordings of at least two wearable devices worn by a user in a specific use case, wherein the specific use case involves a hub executable app being executed on a hub device and designed to measure a characteristic behaviour of the user or designed to perform a surrogate measure of a transient physical characteristic over one or more periods of time according to an embodiment of the present disclosure;
Figure 2 is an illustrative view of different perspectives of two wearable devices for use in the method according to an embodiment of the present disclosure;
Figure 3 is a perspective overview image view of a wearable device and an associated charging cradle for use in the method according to an embodiment of the present disclosure;
Figure 4 is a flow diagram view of a method for synchronizing recordings of at least two wearable devices worn by a user in a specific use case, wherein the specific use case involves a hub executable app being executed on a hub device and designed to measure a characteristic behaviour of the user or designed to perform a surrogate measure of a transient physical characteristic over one or more periods of time according to an embodiment of the present disclosure;
Figure 5 is a flow diagram view of implementing clock-time correction in the method for synchronizing recordings of at least two wearable devices worn by a user in a specific use case designed to measure a characteristic behaviour of the user or designed to perform a surrogate measure of a transient physical characteristic over one or more periods of time according to an embodiment of the present disclosure;
Figure 6 is an illustrative view of two heart rate traces obtained from two wearable devices between the real-time clock hours of 11:00 p.m. on a first day to 11:00 p.m. the following day with a clock difference of one hour between the two heart rate traces according to an embodiment of the present disclosure;
Figure 7 is an illustrative view of the two heart rate traces of Figure 6 that have been magnified to show greater detail of the same according to an embodiment of the present disclosure;
Figure 8 is an illustrative view of a zoomed in section of the two heart rate traces based on Figure 6 which only focuses on the night and wake-up, obtained from two wearable devices between the real-time clock hours of 02:30 a.m. to a few minutes after 11:00 a.m., according to an embodiment of the present disclosure;
Figure 9 is an illustrative view of the two heart rate traces of Figure 8 that have been magnified to show greater detail of the same according to an embodiment of the present disclosure;
Figure 10 is an illustrative view of a zoomed in section of the two heart rate traces based on Figure 8 which only focuses on a part of the night, obtained from two wearable devices between the real-time clock hours of just before 05:35 a.m. to a few minutes after 06:35 a.m., according to an embodiment of the present disclosure;
Figure 11 is an illustrative view of the two heart rate traces of Figure 8 that have been magnified to show greater detail of the same according to an embodiment of the present disclosure; and
Figure 12 is an illustrative view of a dashboard populated with information, collected and/or analyzed, regarding a specific use case in terms of characteristics/conditions under examination (e.g., scratching behaviour) according to an embodiment of the present disclosure.

### DETAILED DESCRIPTION

The embodiments of the present disclosure and the various features and advantageous details thereof are explained more fully with reference to the non-limiting examples that are described and/or illustrated in the drawings and detailed in the following description. It should be noted that the features illustrated in the drawings are not necessarily drawn to scale, and features of one embodiment may be employed with other embodiments as the skilled artisan would recognize, even if not explicitly stated herein. Descriptions of well-known components and processing techniques may be omitted so as to not unnecessarily obscure the embodiments of the present disclosure. The examples used herein are intended merely to facilitate an understanding of ways in which the embodiments of the present may be practiced and to further enable those of skill in the art to practice the same. Accordingly, the examples herein should not be construed as limiting the scope of the present invention, which is defined solely by the appended claims.

It is understood that the embodiments of the present disclosure are not limited to the particular methodology, protocols, devices, apparatus, materials, applications, etc., described herein, as these may vary. It is also to be understood that the terminology used herein is used for the purpose of describing particular embodiments only, and is not intended to be limiting. It must be noted that as used herein and in the appended claims, the singular forms "a," "an," and "the" include plural reference unless the context clearly dictates otherwise.

Unless defined otherwise, all technical and scientific terms used herein have the same meanings as commonly understood by one of ordinary skill in the art to which the embodiments of the present disclosure belong. Preferred methods, devices, and materials are described, although any methods and materials similar or equivalent to those described herein can be used in the practice or testing of the embodiments.

As indicated herein above, for the special use case of monitoring scratching behaviour, patients need to wear two wearable devices (i.e., one device around each wrist) suited with an accelerometer which can measure movements (or accelerations) associated with scratching. In view of the monitored scratching behaviour, a user (or the user's dermatologist or healthcare provider) can be presented with results, such as scratching duration and events. In addition, the results are based upon data that has been collected (via the wearable devices), analyzed and/or processed into scratching behaviour metrics. Furthermore, the data of both wearable devices is used to calculate metrics such as total scratching duration, scratching events and hand usage (left, right, both). Moreover, the clocks of both wearable devices need to be aligned with each other, as well as corrected for clock drift, to enable obtaining of accurate results. A principal element of the method according to the embodiments of the present disclosure is the usage of cardiac activity to align the clocks of two individual wearable devices. This can be achieved with a reflective photoplethysmographic (PPG) sensor that, worn together with the accelerometer used to monitor scratching behaviour, should provide a simultaneous measure of cardiac activity.

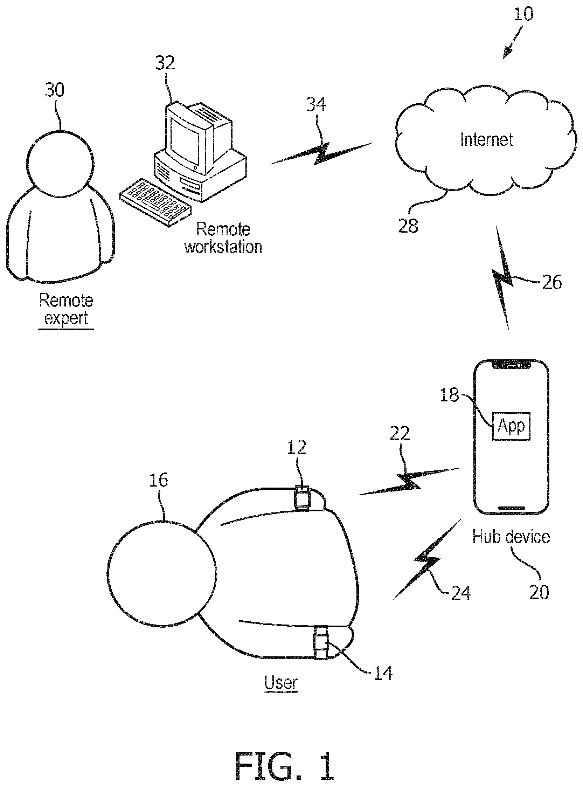

With reference now to Figure 1, a schematic block diagram is illustrated which includes various elements used in a system 10 for synchronizing recordings of at least two wearable devices 12,14 worn by a user 16 in a specific use case. As will be discussed further herein, the specific use case involves a hub executable app 18 being executed on a hub device 20 and designed to measure a characteristic behaviour of the user or designed to perform a surrogate measure of a transient physical characteristic over one or more periods of time according to an embodiment of the present disclosure. The hub device 20 can comprise any suitable smartphone, dedicated hub device, or direct-to-cloud connectivity device having capability to execute the hub executable app 18, as will be discussed further herein. In one embodiment, the characteristic behaviour comprises scratching behaviour, wherein the wearable devices 12,14 are worn one each on a user's wrists. In another embodiment, the transient physical characteristic comprises blood pressure, wherein the wearable devices 12,14 are worn, one on a user's wrist and the other on the upper arm of the same arm as the first wrist worn device.

With reference still to Figure 1, the method comprises acquiring first recordings over one or more periods of time via a first wearable device 12 releasably secured to the user's first wrist. The first wearable device 12 is operable via a first clock (not shown) internal to the first wearable device. The method further comprises acquiring concurrent second recordings via a second wearable device 14 releasably secured to the user, e.g., at the user's second wrist. The second wearable device 14 is operable via a second clock (not shown) internal to the second wearable device. As the first and second wearable devices 12,14 are distinct devices, the first clock is characterized by a first clock drift and offset that may be different from a second clock drift and offset of the second clock.

Subsequent to acquisition of the first and second recordings, the method comprises syncing the first wearable device 12 and the second wearable device 14, via separate syncing operations (illustrated via reference numerals 22 and 24, respectively in Figure 1), with the hub executable app 18 being executed on the hub device 20 for the specific use case. As used herein, syncing comprises transferring respective recordings of each of the first and second wearable devices (12,14) from each respective wearable device to the hub executable app 18 being executed on the hub device 20. Prior to syncing, however, the first wearable device 12 and the second wearable device 14 are paired, via separate distinct, respective pairing operations, with the hub executable app 18 being executed on the hub device 20. As noted herein, the hub device 20 can comprise any suitable smartphone, dedicated hub device, or direct-to-cloud connectivity device configured for executing the hub executable app 18.

According to one embodiment, the wearable devices 12 and 14 comprise Bluetooth^{™} enabled wearable devices configured to sync with the hub executable app 18 executed on the hub device 20, but not with each other. In this manner, the wearable devices are kept to a minimal configuration, without having capability for directly synchronizing clocks between respective wearable devices. In other words, the wearable devices 12 and 14 are separate and distinct from one another, and void of an ability to be directly paired with each other. Alternatively, each wearable device (12, 14) is equipped with means for paired communication with the hub executable app 18 executed on the hub device 20, but not with each other. The means for paired communication can include wired (e.g., via a USB cable or other suitable wiring) or wireless communication. In one example, a Bluetooth^{™} paired wireless communication module (not shown) of the respective wearable device enables paired wireless communication. As indicated, the hub device 20 can comprise any suitable smartphone, dedicated hub device, or direct-to-cloud connectivity device. In yet another embodiment, each wearable device (12, 14) may themselves comprise direct-to-cloud connectivity devices which communicate with the hub executable app 18 being executed in the cloud or internet.

The method further comprises implementing a clock-time correction, specific to the characteristic behaviour being measured or surrogate measure of the transient physical characteristic, of the recordings of both wearable devices 12 and 14 via the hub executable app 18 before processing the respective recordings for the specific use case. The processing of the recordings seeks to obtain an accurate measure of (i) the characteristic behaviour (e.g., scratching behaviour) or (ii) the surrogate measure of the transient physical characteristic (e.g., blood pressure). The clock-time correction advantageiously ensures that the respective recordings have been corrected in time so that a difference between the respective clocks, or clock drift, of the two wearable devices is no more than a threshold duration. The threshold duration is characterized by a shortest event specific to the characteristic behaviour being measured, or the surrogate measure of the transient physical characteristic.

While one special use case described herein relates to scratching behaviour, the embodiments of the present disclosure are also contemplated for other specific use cases. For instance, other relevant use cases are possible where two such wearable device recorders are used, and it is critical to have the wearable device clocks synchronized. One example involves obtaining surrogate measures of (or relative changes in) blood pressure, based on pulse transit time. In such a specific use case, a time difference is calculated between cardiac features from the PPG data (e.g., pulses). A first wearable device with PPG sensor is used on the upper arm, and a second wearable device with PPG sensor is worn at the wrist. Using data from the first and second wearable devices, an estimate of time difference between cardiac features can be obtained. This use case would require a very precise global (as in, for the entire recording) synchronization of the two clocks. Accordingly, the embodiments of the present disclosure can cover cases where two recorders are utilized to capture a given transient behavior or changing physical property, and where synchronization is critical for the proper estimation of that behavior/measure.

With reference still to Figure 1, in one embodiment, the hub executable app 18 executed on the hub device 20 is further configured for connectivity (e.g., wireless or wired, illustrated via reference numeral 26) to the internet 28. The hub executable app 18 may also be configured for communicating, via the internet 28, with a remote expert 30, via a remote workstation 32, wherein the remote workstation is coupled to the internet via wireless or wired connectivity indicated via reference numeral 34.

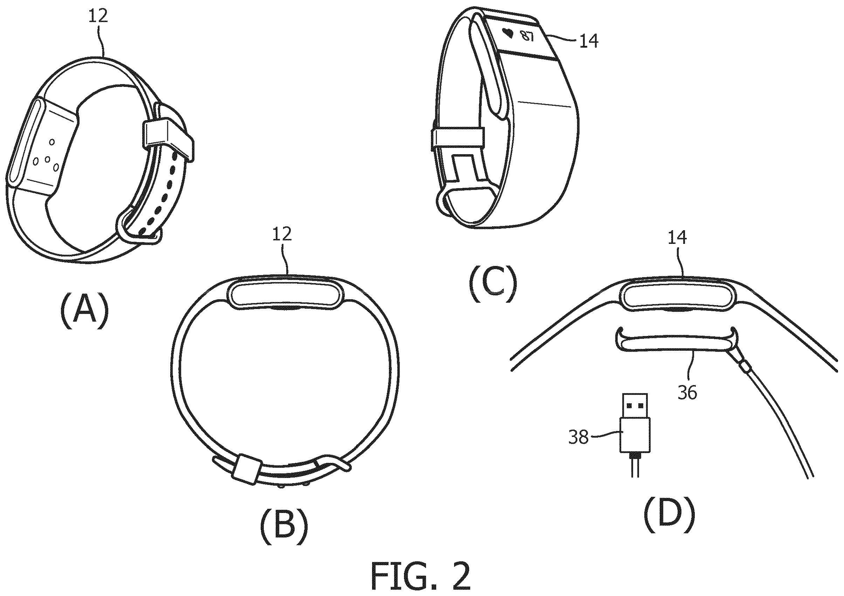

Turning now to Figure 2, there is shown an illustrative view of different perspectives of two wearable devices 12 and 14 for use in the method according to an embodiment of the present disclosure. The wearable devices 12 and 14 can each comprise a health band (e.g., DL7421, DL7422 Philips health band commercially available from Philips Consumer Lifestyle, a division of Philips North America LLC, Stamford, CT) which measures, tracks, and analyzes movement and physiological parameters. These metrics are intended as input for behavioral change programs for managing lifestyle of individuals with risk of or affected by weight and activity related chronic disease. Investigation of these parameters is used in the assessment of the physiological state of a person and their daily physical activity level. The continuous monitoring of these parameters can give insight in changes of heart rate and daily physical activity. The wearable device is intended for 24/7 use in daily life, both indoor and outdoor. However, a given special use case may only require both wearable devices 12 and 14 to be worn for a period of time less than 24/7, for example, during periods of sleep, from retiring in the evening until awakening the next morning, for one or more consecutive days for the duration of the special use case implementation.

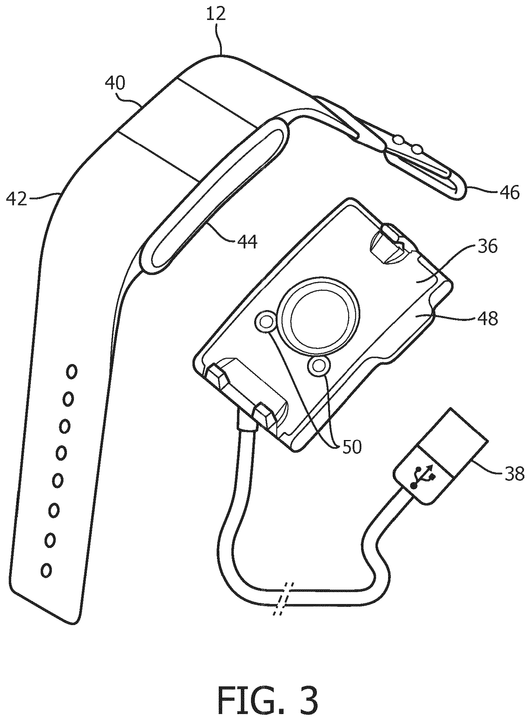

With reference now to Figure 3, there is shown a perspective overview image view of a wearable device 12 and an associated charger 36 (or charging cradle with an associated connector 38) for use in the method according to an embodiment of the present disclosure. In the overview image of Figure 3, the wearable device 12 comprises a display 40, wristband 42, housing 44, and a buckle 46. Charger 36 includes a release tab 48, a set of charging pins 50 and a USB connector 38.

Referring now to Figures 1, 2 and 3, the basic operating principle of each individual health band is as follows. The health band is a wrist-worn activity (accelerometer) and heart rate measurement device that uses the Opticardiogram technology. The general operating principle of the Opticardiogram technology is based on light-emitting diodes (LEDs) emitting light through the surface of the skin. Part of that light is reflected and collected by the photo sensor. The blood flow in the arteries is driven by the heart beating and pumping blood. The blood flow changes over time. Therefore, the amount of light reflected varies over time. The sensor in the health band collects this change in reflection of light. The health band enables a user to develop a healthier lifestyle by tracking his/her heart rate, activity and sleep with medical accuracy. All recorded measurements or statistics are synced via Bluetooth^{®} with a given health app, the health app operating on a compatible smartphone or hub device, where the health band user can find details of health metrics, as well as progress towards personal targets or goals.

A wearable device user follows user instructions when initially setting up each wearable device with the hub executable app 18. The user instructions include at least filling in correct user profile data, and wearing side (e.g., left wrist, right-hand wrist) per wearable device.

In initial preparation for use, a user first downloads the hub executable app 18 to the user's smartphone (or hub device) from an appropriate app store, e.g., the App Store or Google Play, wherein the user's smartphone is first determined to be compatible with the app. If the hub executable app 18 is already installed on the user's smartphone (i.e., for a first connected wearable device 12), then the user goes to the settings of the hub executable app 18 and adds a second wearable device 14 (health band) as a new device. The hub executable app 18 is then executed, and the user follows the setup instructions in the hub executable app to setup the respective health band (12,14) and pair it with his/her smartphone (or hub device 20). Providing correct profile data in the hub executable app 18 is important to enable the obtaining of accurate health metrics via a respective health band/wearable device, 12 or 14.

During pairing of each health band/wearable device (12, 14) with the smartphone 20, the hub executable app 18 is active and Bluetooth^{™} and internet connectivity are enabled on the smartphone. In addition, during pairing, the smartphone and each respective health band/wearable device (12, 14) are physically kept within syncing range (i.e., no more than 2 meters / 6 feet from each other, in the same room). When each health band/wearable device (12, 14) is successfully paired with the smartphone 20, the firmware of the respective health band can be updated to match local time, profile settings and possibly other features. In addition, responsive to being successfully paired, the respective health band (12, 14) can automatically sync the user's personal health data to the smartphone 20 via Bluetooth^{™}. In one embodiment, each respective health band (12, 14) is activated by tapping on the respective device's display 40 twice.

With respect to the syncing and storing of personal health data, each health band (12, 14) is equipped with Bluetooth^{™} which enables the syncing of the user's personal health data from each respective health band to the user's smartphone 20. The user's personal health data is then stored, e.g., in a cloud-based platform and shown in the hub executable app 18, according to the specifics of a given use-case implementation. Syncing of the user's personal health data is accomplished via first opening the hub executable app 18 and activating Bluetooth^{™} on the user's smartphone 20. The smartphone and each respective health band are kept within syncing distance from each other. Once successfully connected, the measurement results from each respective health band are synced to the hub executable app 18. If the sync is successful, an indication may be displayed in the hub executable app 18, wherein the user may check personal health data on the smartphone 20 via the hub executable app.

In further discussion of each wearable device, the heart rate sensor of each device captures raw PPG data (i.e., unprocessed), which can be translated into metrics such as average heart rate and inter-beat intervals. That is, raw (i.e., unprocessed) PPG data can be translated into metrics of average heart rate and inter-beat intervals using suitable techniques known in the art. Where the accelerometer signal of both wearable devices is different (because they originate from the different wrist), the heart rate (or heart rate derived features) will be rather similar. There is a chance that some values of the heart rate signal might actually be different, due to signal processing and the influence of the accelerations on the heart rate signal. However, considering the "big picture", heart rate or heart rate derived features should be the same for both wearable devices since the origin of both is with the same heart.

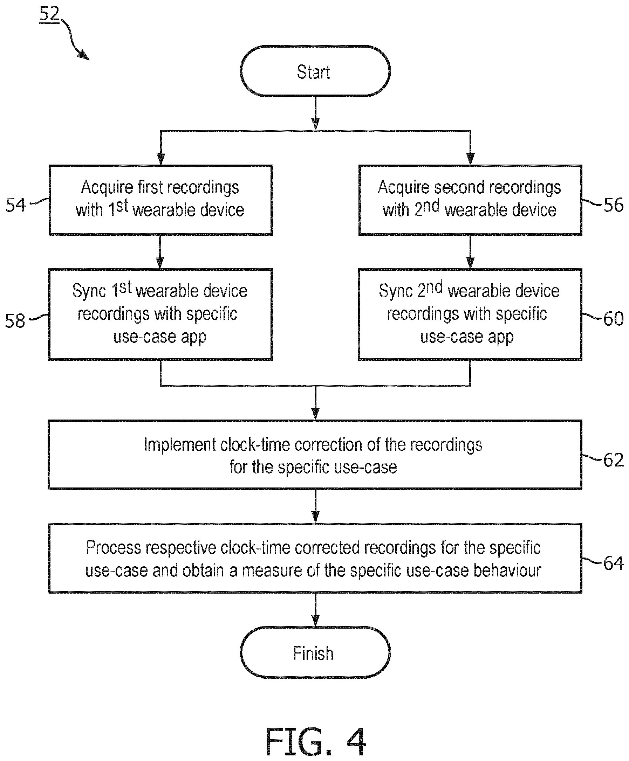

With reference now to Figure 4, there is shown a flow diagram view of a method 52 for synchronizing recordings of at least two wearable devices worn by a user in a specific use case designed to measure a characteristic behaviour (e.g., scratching behaviour) of the user over one or more periods of time (e.g., during periods of sleep). As discussed herein, the specific use case includes executing the hub executable app 18 on a hub device 20. Method 52 comprises a first Step 54 of acquiring first recordings via the first wearable device 12 releasably secured to the user's first wrist, wherein the first wearable device is operable via a first clock internal to the first wearable device. In a concurrent Step 56, the method comprises acquiring concurrent second recordings via the second wearable device 14 releasably secured to the user, e.g., at the user's second wrist. The second wearable device is operable via a second clock internal to the second wearable device. It is noted that, given the first and second wearable devices are distinct separate devices, the first clock can be characterized by a first clock drift and offset that may be different from a second clock drift and offset of the second clock. In one embodiment, the first recordings comprise at least accelerometer and photoplethysmography (PPG) data as a function of time per the first clock, and wherein the second recordings comprise at least accelerometer and photoplethysmography (PPG) data as a function of time per the second clock.

The method continues with syncing the first wearable device 12 and the second wearable device 14, via separate syncing operations (Steps 58 and 60, respectively), with the hub executable app 18 being executed on the hub device 20 for the specific use case. For syncing and storing of data, in one embodiment, each wearable device (12, 14) is equipped with Bluetooth^{™} which enables the syncing of the recordings from each respective wearable device (12, 14) to the hub device 20. The recordings data is then stored, e.g., in a cloud-based platform and shown in the hub executable app 18, according to the specifics of the given use-case implementation. Syncing of the recordings data is accomplished via first opening the hub executable app 18 and activating Bluetooth^{™} on the hub device 20. The hub device 20 and each respective wearable device (12, 14) are kept within syncing distance from each other. Once successfully connected, the recordings or measurement results from each respective wearable device or health band are synced to the hub executable app 18. If the sync is successful, an indication may be displayed in the hub executable app 18, wherein the user may check corresponding recordings and/or personal health data on the hub device 20 via the hub executable app 18.

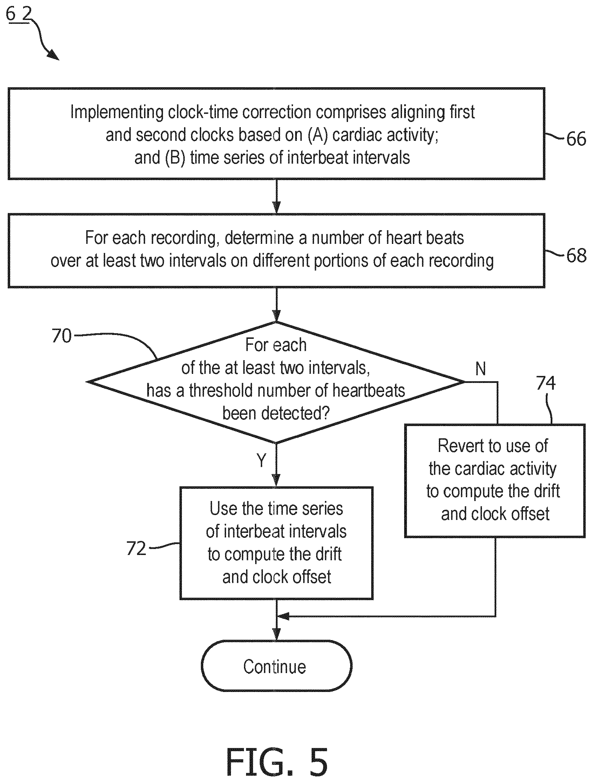

With reference still to Figure 4, the method 52 further comprises implementing a clock-time correction, specific to the characteristic behaviour being measured, of the recordings of both wearable devices via the hub executable app (Step 62), as will be further discussed herein with reference to Figure 5. Method 52 still further comprises processing the respective recordings for the specific use case (Step 64) to obtain a measure of the characteristic behaviour (e.g., scratching behaviour). The clock-time correction advantageously ensures that the respective recordings have been corrected in time so that a difference between the respective clocks, or clock drift, of the two wearable devices (12, 14) is no more than a threshold duration of a shortest event specific to the characteristic behaviour being measured.

With reference now to Figure 5, there is shown a flow diagram view of implementing clock-time correction (Step 62, Figure 4) in the method for synchronizing recordings of at least two wearable devices according to an embodiment of the present disclosure, the devices being worn by the user in the specific use case designed to measure a characteristic behaviour of the user over one or more periods of time. In Step 66, implementing the clock-time correction comprises aligning the first and second clocks based on (A) cardiac activity that includes a heart rate derived feature (e.g., an average heart rate) based on photoplethysmography (PPG) data detected via a heart rate sensor (i.e., a PPG sensor) of each respective wearable device, and (B) a time series of interbeat intervals derived from PPG data detected via the PPG sensor of each respective wearable device.

In Step 68, the method includes determining, as a function of a given length of the recordings, a number of heart beats over at least two different intervals of predetermined duration on each of at least two different portions of the recordings from each respective wearable device. The determination is to include at least one proximal portion near a beginning and one distal portion near an ending of each respective recording of the two recordings. In other words, the length of the recording has implications on the amount of clock drift that can be expected. In any case, to correct clock drift it is more important to have a sufficient number of heart beats (e.g., covering an interval of 5-15 minutes) detected on different parts of the recording (e.g., at the beginning and at the end) than it is to have a higher percentage of heart beats all concentrated around a single point.

For the specific use case of scratching behaviour, scratching behavior is to be monitored while the patient is at rest/asleep, during the night. As such, a typical recording duration is the full night, and the embodiments of the present disclosure can be applied to synchronize the clocks of the two wearable devices on that specific recording period. Even if the recording is repeated on successive nights, one logical approach would be to align the clocks night per night (although certainly not excluding the possibility of aligning the clocks with the data of multiple recording nights - ignoring the daytime parts of the recording, that is).

In Step 70, for each of the at least two intervals, the method queries whether a threshold number of heart beats, over the at least two different intervals of predetermined duration, have been detected on each of the at least two different portions. If so, then the method advances to Step 72, using the time series of interbeat intervals to compute the drift and clock offset. Otherwise, the method at Step 74 reverts to use of the cardiac activity to compute the drift and clock offset.

In Step 72, the clock-time correction based on the time series of interbeat intervals comprises the following sequence of steps: (1) For each recording, automatically detect and represent (a) temporal locations of heart beats on each PPG signal trace and (b) interbeat intervals as two non-equally sampled time series, each sample in each time series indicating a pair that comprises (time, interbeat interval), wherein "time" corresponds to a clock time (temporal location) of each detected heartbeat, and "interbeat interval" corresponds to a time distance to a next heart beat or a previous heartbeat. (2) Interpolate each time series to a fixed sampling rate 'fs'. This can be performed using any suitable method, for example, with linear interpolation. (3) Select one of the two PPG signal traces from the recordings of the respective wearable devices as a "master" and use the clock of the wearable device which corresponds with the selected master PPG signal trace as a master clock that from then on will be used as a reference for absolute time. (4) Synchronize the offset and clock drift between the two interpolated time series signals using a suitable method such as described further herein below.

For example, synchronization of the offset and clock drift comprises sub-steps as follows: (a) finding a delay, whether positive or negative, of the "slave" signal that maximizes a correlation between the two interpolated time series signals, wherein finding the delay comprises finding a delay that gives a maximum cross-correlation between the two interpolated time series signals; (b) applying a multiplication factor to the fixed sampling rate 'fs' to compensate for whatever drift the slave clock might have; and (c) repeating steps (a) and (b) in search of the multiplication factor, within a specified time interval, to find an optimal multiplication factor and delay that gives an overall highest cross-correlation between the two interpolated time series signals. Furthermore, the method comprises applying the optimal multiplication factor (clock drift) and delay (offset) to the accelerometer signal of the slave device to obtain two accurately synchronized recordings of acceleration, which can thereby be used to process the respective recordings for the specific use case to obtain the measure of the characteristic behaviour (e.g., scratching behaviour) and/or detect given use case events (e.g., scratching events).

In Step 74, the clock-time correction based on cardiac activity comprises aligning a heart rate derived feature trace (e.g., an average heart rate trace) of the first recordings with a heart rate derived feature trace (e.g., an average heart rate trace) of the second recordings via shifting one trace in time with respect to the other trace by an amount such that an amplitude of the heart rate derived feature traces (i.e., average heart rate traces) of the first and second recordings overlap and match within a given threshold percentage. Such a threshold percentage is dependent upon the particular specific use case implementation. If the overlap and match is at or above the threshold percentage, then the corresponding shifting is accepted for the clock-time correction; otherwise, below the threshold percentage is rejected as not acceptable for the clock-time correction.

With respect to the two approaches of clock-time correction based on (i) using time series of interbeat intervals and (ii) cardiac activity, the benefit of using the time series of interbeat intervals over using cardiac activity is that the time series of interbeat intervals provides a more accurate alignment of the two clocks, in particular when the recording is not very long, or when the recording contains too many artefacts. A disadvantage of using the time series of interbeat intervals over using cardiac activity is that the detection of individual heart beats is more sensitive to motion artefacts (e.g., during the actual scratching events) than average heart rate, which may complicate the synchronization, for example when the user moves during most of the recording. An additional option could be to consider a combination of using time series of interbeat intervals (or cardiac activity) and a different modality (e.g., body movements as captured by accelerometer or actigraphy) in an extension of the embodiments of the present disclosure. Such an embodiment could be made more robust by using body movements to roughly align the two clocks based on general body movements of the entire body (or simultaneously of the two wrists), and then further tune the alignment based on either cardiac activity method (i.e., using time series of interbeat intervals or cardiac activity).

In one embodiment, alignment between two clocks is done by calculating the clock offset and the clock drift between the two. To determine the clock offset, it is sufficient to have enough valid "overlapping" (between the two wearable device recorders) data, say one (1) hour (but certainly not limited to this, it can probably be achieved with less, provided there's enough variation). To determine the drift, however, although it can still be done with such a segment, ideally it is desirable to have as much time difference between several points in the analysis (to cause the largest drift possible). So, taking a period of valid heart rates/heart beats at the beginning of the recording (e.g., 5-15 minutes at the beginning of the resting period - so when the body movements ceased and therefore the quality of the PPG data increased), and close to the end of the recording (e.g., 5-15 minutes around the end of the resting period, for instance in morning), should be sufficient to calculate the clock drift between the two clocks of the respective wearable devices. Obviously, this is not the only way to achieve this purpose, as other methods are also feasible. In a more advanced embodiment, where there are multiple recordings of several nights, one could actually further improve the estimates of clock drift by combining and further improving this estimation based on the analysis of different recordings, since clock drift should remain (i.e., under stable temperature conditions) relatively stable from one night to the next.

In one embodiment, the specific use case comprises monitoring scratching behaviour and measuring movements associated with scratching via the first and second wearable devices. As appropriate, the recordings of both wearable devices are used to calculate metrics which include one or more of total scratching duration, scratching events and hand usage. The recordings of the first and second wearable devices comprise twenty hertz (20 Hz) data for a one night synchronization. The duration of the shortest scratching event is on the order of two seconds (2 s), and the threshold duration is no more than one second (1 s). In other words, a reasonable threshold for clock drift is 50% of the shortest possible scratching event so as to guarantee that at least half of the scratching events detected on the left-hand wrist and right-hand wrist overlap and that the events are therefore correctly classified, as for example, both hand scratching. In the special use case of scratching behaviour, a synchronization of the clocks for each sleep/resting period (overnight) can be performed separately, as necessary, and not for multiple nights all at once.

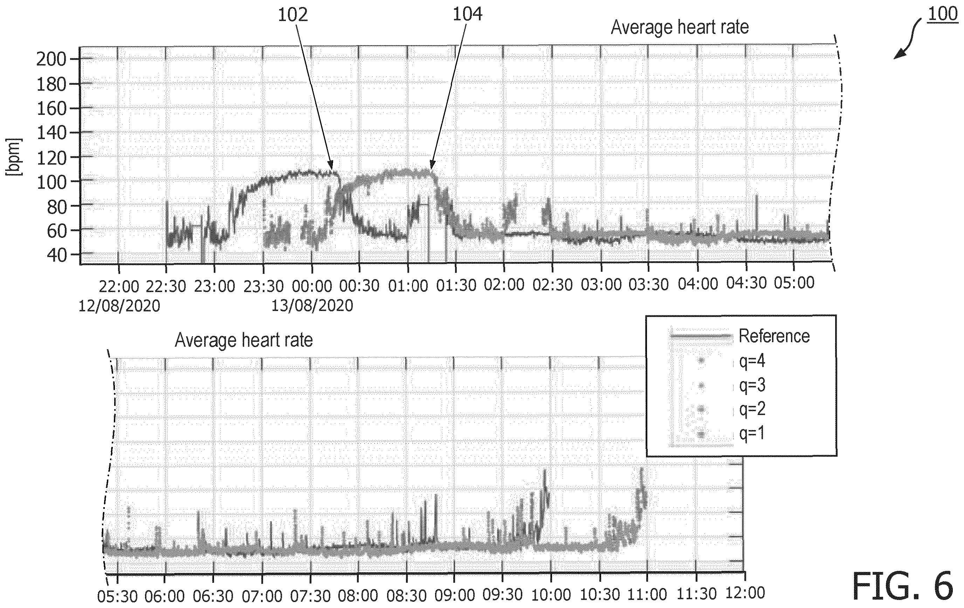

With reference now to Figure 6, there is shown an illustrative view 100 of two heart rate traces 102 and 104 obtained from two wearable devices 12 and 14, in units of beats-per-minute (bpm), between the real-time clock hours of 11:00 p.m. on a first day to 11:00 p.m. the following day with a clock difference of one hour between the two heart rate traces according to an embodiment of the present disclosure. In other words, Figure 6 gives an example of two heart rate traces (i.e., raw PPG data collected over a period of time), which included a session of physical activity at the beginning of the recording. Visually, it is clear how the trace signals should be shifted in time such that the two recordings overlap (i.e., so that the two recordings overlap (and are synchronized with) one another).

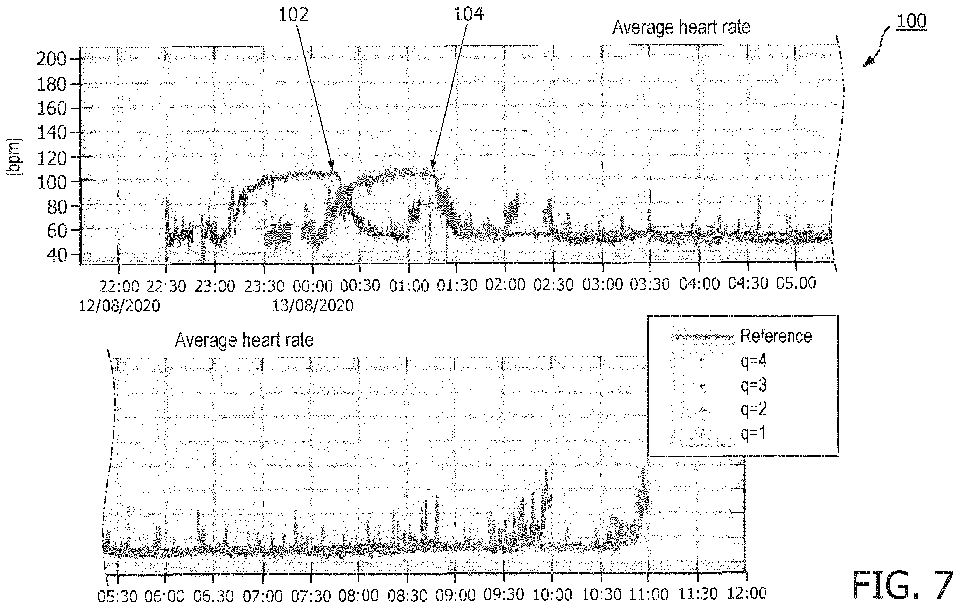

Turning our attention now to Figure 7, there is shown an illustrative view 100 of the two heart rate traces 102 and 104 of Figure 6 that have been magnified to show greater detail of the same according to an embodiment of the present disclosure. The two heart rate traces have a clock difference of exactly one (1) hour. Synchronization of the two heart traces can be accomplished, for example, via a visual alignment.

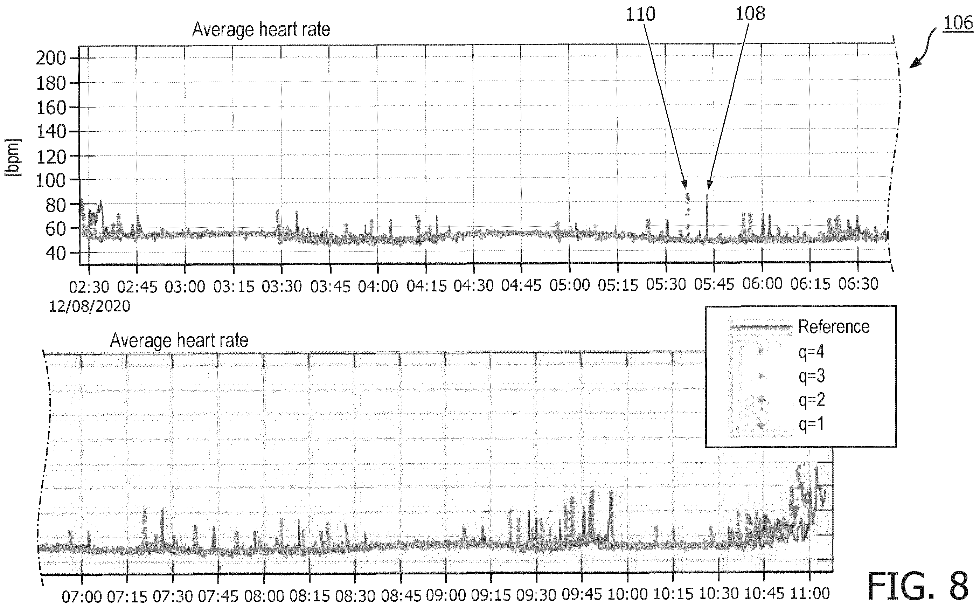

With reference now to Figure 8, there is shown is an illustrative view 106 of a zoomed in section of two heart rate traces 108 and 110 based on Figure 6 which only focuses on the night and wake-up, obtained from two wearable devices, in units of beats-per-minute (bpm), between the real-time clock hours of 02:30 a.m. to a few minutes after 11:00 a.m., according to an embodiment of the present disclosure. The high, sudden heart rate peaks are caused by arousals and provide an excellent means of syncing both signals (i.e., syncing the recordings of both wearable devices with each other).

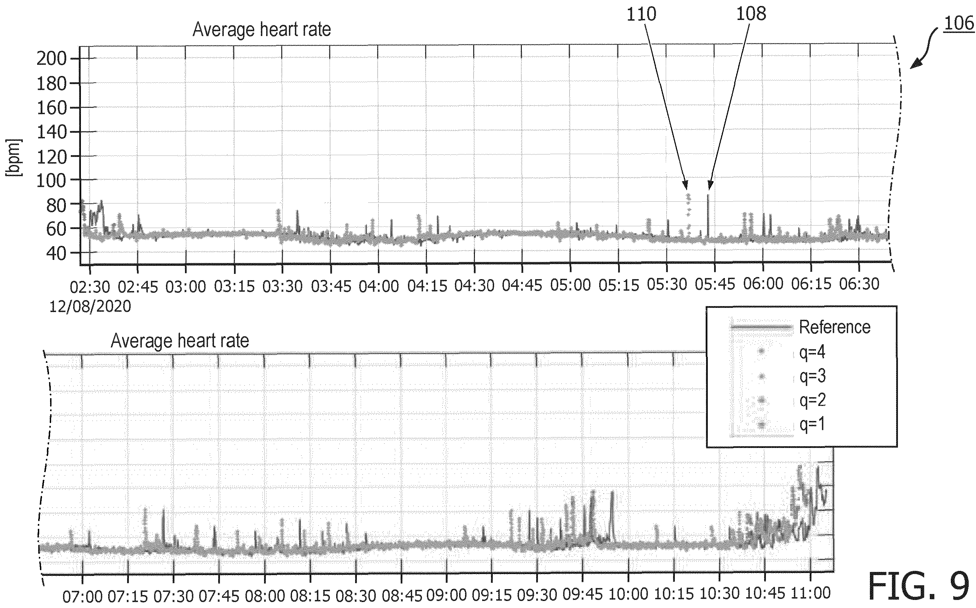

With reference now to Figure 9, there is shown an illustrative view 106 of the two heart rate traces 108 and 110 of Figure 8 that have been magnified to show greater detail of the same according to an embodiment of the present disclosure. In practice, both traces are considered in their entirety and a suitable algorithm is used to find the optimal fit, taking into account that between the two wearable devices the estimated cardiac activity features might vary (every measurement has an error). The best fit is used.

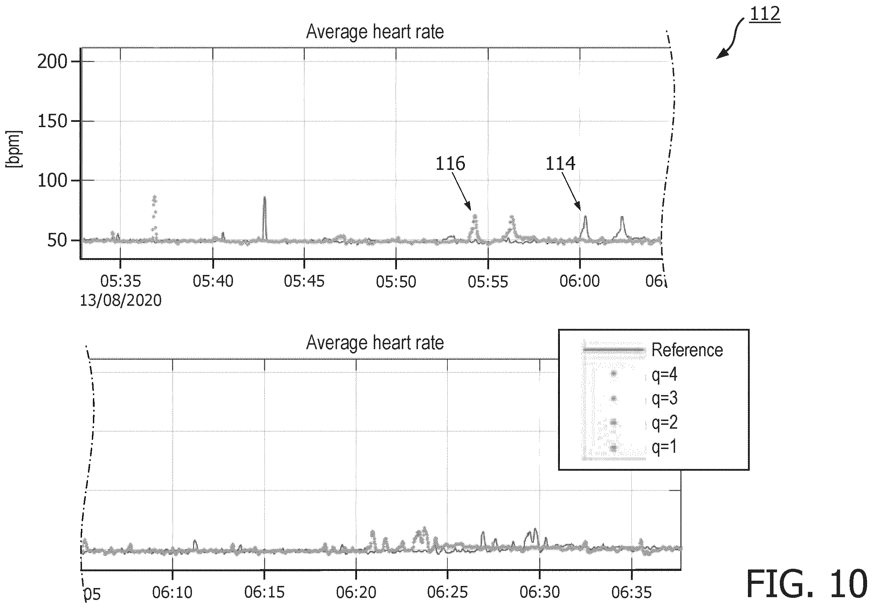

Turning now to Figure 10, there is shown an illustrative view 112 of a zoomed in section of the two heart rate traces 114 and 116 based on Figure 8 which only focuses on a part of the night, obtained from two wearable devices, in units of beats-per-minute (bpm) between the real-time clock hours of just before 05:35 a.m. to a few minutes after 06:35 a.m., according to an embodiment of the present disclosure. Similar with the trace magnification in Figure 8, the high, sudden heart rate peaks in Figure 10 are also caused by arousals and provide a further excellent means of syncing both signals on a more granular scale (e.g., within 5 seconds), i.e., synchronizing the recordings of both wearable devices with each other.

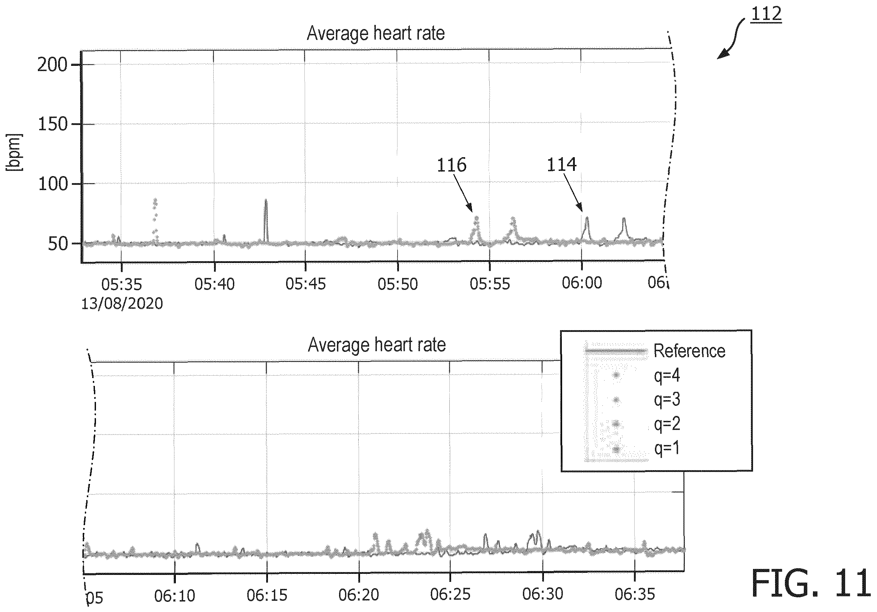

With reference now to Figure 11, there is shown an illustrative view 112 of the two heart rate traces 114 and 116 of Figure 8 that have been magnified to show greater detail of the same according to an embodiment of the present disclosure.

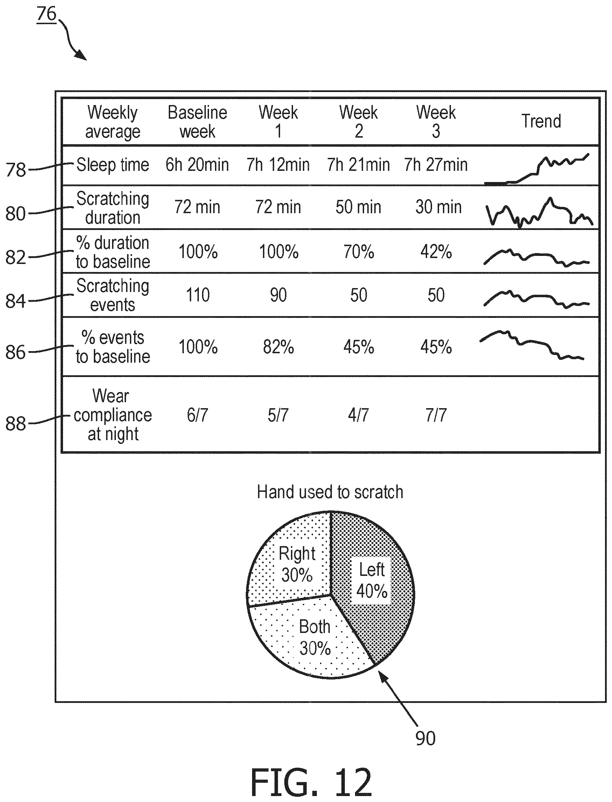

Turning now to Figure 12, there is shown an illustrative view of a dashboard 76 populated with information, collected and/or analyzed, regarding a specific use case in terms of characteristics/conditions under examination (e.g., scratching behaviour) according to an embodiment of the present disclosure. In particular, the hub executable app 20 (Figure 1) is configured to generate the dashboard 76 of the specific use case information based on collected and analyzed data regarding characteristics/conditions under examination over at least one predetermined duration of time. As illustrated, the specific use case comprises scratching behaviour. A scratching algorithm analyzes the synchronized data and outputs a list with scratching events (start time, duration, left/right/both hands, mean power), and all other endpoints are aggregated from this, like total scratching duration, number of events, etc. The dashboard 76 includes a weekly average of one or more of sleep time 78, scratching duration 80, percentage scratching duration to baseline 82, scratching events 84, percentage scratching events to baseline 86, and wear compliance at night 88, shown in a listing on the left-hand side of the dashboard 76. The weekly average data collected via the recordings populates the dashboard, as indicated by examples, for each of a baseline week, week 1, week 2, week 3, and a trend graph. Labels across the top of the dashboard, from left to right, indicate a specific week or trend. The trend graphs provide a graphical trend summary for a corresponding weekly average sleep time, scratching duration, percentage scratching duration to baseline, scratching events, percentage scratching events to baseline, and wear compliance at night. The dashboard further includes a percentage indicator 90 of respective usage of left-hand, right-hand and both left- and right-hand for the scratching behavior. In the example shown, right-hand scratching was determined to be 30% of the time, left-hand scratching 40% of the time, and both hand scratching 30% of the time.

In one embodiment, the hub executable app 18 can comprise one or more modules that comprise one or more of an integrated circuit, discrete analog or digital circuit components, hardware, software, firmware, or any combination thereof, for performing various functions as discussed herein, further according to the requirements of a given special use case implementation and/or application. In addition, one or more of the modules can further comprise various combinations of one or more of the various modules.

In one embodiment, a controller implemented within the hub device 20 comprises one or more of a microprocessor, microcontroller, field programmable gate array (FPGA), integrated circuit, discrete analog or digital circuit components, hardware, software, firmware, or any combination thereof, for performing various functions as discussed herein, further according to the requirements of a given special use case device implementation and/or application. The controller can further comprise one or more of the various modules as discussed herein.

It is understood that the modules described herein of the special use case implementation may be computer program modules which are rendered in a non-transitory computer-readable medium. Accordingly, the non-transitory computer readable medium is embodied with instructions executable by a processor for causing the processor to carry out the method according to the various embodiments of the present disclosure, for synchronizing recordings of at least two wearable devices worn by the user in the specific use case designed to measure the characteristic behaviour of the user or designed to perform a surrogate measure of a transient physical characteristic over one or more periods of time.

Although only a few exemplary embodiments have been described in detail above, those skilled in the art will readily appreciate that many modifications are possible in the exemplary embodiments without materially departing from the novel teachings and advantages of the embodiments of the present disclosure. Accordingly, all such modifications are intended to be included within the scope of the embodiments of the present disclosure.

In addition, any reference signs placed in parentheses in one or more claims shall not be construed as limiting the claims. The word "comprising" and "comprises," and the like, does not exclude the presence of elements or steps other than those listed in any claim or the specification as a whole. The singular reference of an element does not exclude the plural references of such elements and vice-versa. One or more of the embodiments may be implemented by means of hardware comprising several distinct elements, and/or by means of a suitably programmed computer. In a device claim enumerating several means, several of these means may be embodied by one and the same item of hardware. The mere fact that certain measures are recited in mutually different dependent claims does not indicate that a combination of these measures cannot be used to an advantage.

## Claims

1. A method for synchronizing recordings of at least two wearable devices worn by a user in a specific use case, wherein the specific use case involves a hub executable app being executed on a hub device and designed to measure a characteristic behaviour of the user or designed to perform a surrogate measure of a transient physical characteristic over one or more periods of time, the method comprising:
acquiring first recordings via a first wearable device, the first wearable device operable via a first clock internal to the first wearable device;
acquiring concurrent second recordings via a second wearable device, the second wearable device operable via a second clock internal to the second wearable device, and wherein the first clock is **characterized by** a first clock drift and offset that may be different from a second clock drift and offset of the second clock;
syncing the first wearable device and the second wearable device, via separate syncing operations, with the hub executable app being executed on the hub device for the specific use case; and
implementing a clock-time correction, specific to the characteristic behaviour being measured or surrogate measure of the transient physical characteristic, of the recordings of both wearable devices via the hub executable app before processing the respective recordings for the specific use case to obtain a measure of the characteristic behaviour or the surrogate measure of the transient physical characteristic, wherein the clock-time correction ensures that the respective recordings have been corrected in time so that a difference between the respective clocks, or clock drift, of the two wearable devices is no more than a threshold duration of a shortest event specific to the characteristic behaviour being measured or the surrogate measure of the transient physical characteristic,
wherein the first recordings comprise at least accelerometer and photoplethysmographic (PPG) data as a function of time per the first clock, and wherein the second recordings comprise at least accelerometer and photoplethysmographic (PPG) data as a function of time per the second clock,
**characterized in that** implementing the clock-time correction comprises aligning the first and second clocks based on a time series of interbeat intervals derived from PPG data detected via a PPG sensor of each respective wearable device,
wherein the clock-time correction based on the time series of interbeat intervals further comprises:
(1) for each recording, automatically detect and represent (a) temporal locations of heart beats on each PPG signal trace and (b) interbeat intervals as two non-equally sampled time series, each sample in each time series indicating a pair that comprises (time, interbeat interval), wherein "time" corresponds to a clock time or temporal location of each detected heart beat, and "interbeat interval" corresponds to a time distance to a next heart beat or a previous heart beat;
(2) interpolate each time series to a fixed sampling rate 'fs';
(3) select one of the two PPG signal traces from the recordings of the respective wearable devices as a "master" and use the clock of the wearable device which corresponds with the selected master PPG signal trace as a master clock that from then on will be used as a reference for absolute time; and
(4) synchronize the offset and clock drift between the two interpolated time series signals.

2. The method according to claim 1, wherein synchronization of the offset and clock drift comprises:
(a) finding a delay, whether positive or negative, of the "slave" signal that maximizes a correlation between the two interpolated time series signals, wherein finding the delay comprises finding a delay that gives a maximum cross-correlation between the two interpolated time series signals;
(b) applying a multiplication factor to the fixed sampling rate 'fs' to compensate for whatever drift the slave clock might have; and
(c) repeating steps (a) and (b) in search of the multiplication factor, within a specified time interval, to find an optimal multiplication factor and delay that gives an overall highest cross-correlation between the two interpolated time series signals.

3. The method according to claim 2, further comprising:
applying the optimal multiplication factor, relating to clock drift, and delay, relating to offset, to the accelerometer signal of the slave device to obtain two accurately synchronized recordings of acceleration, which can thereby be used to process the respective recordings for the specific use case to obtain the measure of the characteristic behaviour and/or detect given use case events.

4. The method according to claim 1, wherein prior to syncing, the method further comprises pairing the first wearable device and the second wearable device, via separate pairing operations, with the hub executable app being executed on the hub device, wherein the hub device comprises at least one of a smartphone, a dedicated hub device, and direct-to-cloud connectivity device.

5. The method according to claim 1, wherein the wearable devices comprise Bluetooth^{™} enabled wearable devices configured to sync with the hub executable app executed on the hub device, but not with each other.

6. The method according to claim 1, wherein each wearable device is equipped with means for paired wireless communication with the hub executable app executed on the hub device, but not with each other.

7. The method according to claim 6, wherein the means for paired wireless communication comprises a Bluetooth^{™} paired wireless communication module of the respective wearable device, and wherein the hub device comprises at least one of a smartphone, a dedicated hub device, and direct-to-cloud connectivity device.

8. The method according to claim 1, wherein syncing comprises transferring respective recordings of each of the first and second wearable devices from each respective wearable device to the hub executable app being executed on the hub device.

9. A hub device configured to execute a hub executable app pursuant to the method according to claim 1, for synchronizing recordings of at least two wearable devices worn by a user in a specific use case, wherein the specific use case involves the hub executable app being executed on the hub device and designed to measure a characteristic behaviour of the user or designed to perform a surrogate measure of a transient physical characteristic over one or more periods of time.

10. A non-transitory computer readable medium embodied with instructions executable by a processor for causing the processor to carry out the method according to claim 1, for synchronizing recordings of at least two wearable devices worn by the user in the specific use case designed to measure the characteristic behaviour of the user or designed to perform a surrogate measure of a transient physical characteristic over one or more periods of time.

## Patentansprüche

1. Verfahren zum Synchronisieren von Aufzeichnungen von mindestens zwei tragbaren Vorrichtungen, die von einem Benutzer in einem speziellen Verwendungsfall getragen werden, wobei der spezielle Verwendungsfall einbezieht, dass eine Hub-ausführbare App auf einer Hub-Vorrichtung ausgeführt wird, die dazu ausgelegt ist, ein charakteristisches Verhalten des Benutzers zu messen, oder dazu ausgelegt ist, eine Ersatzmessung einer vorübergehenden physikalischen Eigenschaft über einen oder mehrere Zeiträume durchzuführen, wobei das Verfahren umfasst:
Erfassen erster Aufzeichnungen über eine erste tragbare Vorrichtung, wobei die erste tragbare Vorrichtung über eine erste Uhr betreibbar ist, die sich in der ersten tragbaren Vorrichtung befindet;
Erfassen gleichzeitiger zweiter Aufzeichnungen über eine zweite tragbare Vorrichtung, wobei die zweite tragbare Vorrichtung über einen zweiten Takt innerhalb der zweiten tragbaren Vorrichtung betreibbar ist, und wobei der erste Takt durch eine erste Taktabweichung und einen Versatz gekennzeichnet ist, die sich von einer zweiten Taktabweichung und einem Versatz des zweiten Takts unterscheiden können;
Synchronisieren der ersten tragbaren Vorrichtung und der zweiten tragbaren Vorrichtung über separate Synchronisierungsvorgänge mit der Hub-ausführbaren App, die auf der Hub-Vorrichtung für den speziellen Verwendungsfall ausgeführt wird; und
Implementieren einer Uhrzeitkorrektur, die spezifisch für das charakteristische Verhalten ist, das gemessen wird, oder für die Ersatzmessung der vorübergehenden physikalischen Eigenschaft, der Aufzeichnungen beider tragbarer Vorrichtungen über die Hub-ausführbare App, bevor die jeweiligen Aufzeichnungen für den speziellen Verwendungsfall verarbeitet werden, um eine Messung des charakteristischen Verhaltens oder die Ersatzmessung der vorübergehenden physikalischen Eigenschaft zu erhalten, wobei die Uhrzeitkorrektur sicherstellt, dass die jeweiligen Aufzeichnungen zeitlich korrigiert wurden, so dass eine Differenz zwischen den jeweiligen Uhren, oder die Uhrabweichung, der beiden tragbaren Vorrichtungen nicht mehr als eine Schwellendauer eines kürzesten Ereignisses beträgt, das für das gemessene charakteristische Verhalten oder die Ersatzmessung der vorübergehenden physikalischen Eigenschaft spezifisch ist, wobei die ersten Aufzeichnungen mindestens Beschleunigungsmesser und photoplethysmographische (PPG) Daten als Funktion der Zeit pro erste Uhr umfassen, und wobei die zweiten Aufzeichnungen mindestens Beschleunigungsmesser und photoplethysmographische (PPG) Daten als Funktion der Zeit pro zweite Uhr umfassen,
**dadurch gekennzeichnet, dass** Implementieren der Uhrzeitkorrektur Ausrichten des ersten und zweiten Takts auf der Grundlage einer Zeitreihe von Zwischenschlagintervallen umfasst, die aus PPG-Daten abgeleitet werden, die über einen PPG-Sensor jeder jeweiligen tragbaren Vorrichtung erkannt werden,
wobei die auf der Zeitreihe der Zwischenschlagintervalle basierende Uhrzeitkorrektur weiter umfasst:
(1) für jede Aufzeichnung, automatisches Erkennen und Darstellen (a) zeitlicher Positionen von Herzschlägen auf jeder PPG-Signalspur und (b) der Zwischenschlagintervalle als zwei ungleichmäßig abgetastete Zeitreihen, wobei jede Abtastung in jeder Zeitreihe eine Kopplung anzeigt, das (Zeit, Zwischenschlagintervall) umfasst, wobei "Zeit" einer Uhrzeit oder einer zeitlichen Position jedes erkannten Herzschlags entspricht, und "Zwischenschlagintervall" einem zeitlichen Abstand zu einem nächsten Herzschlag oder zu einem vorherigen Herzschlag entspricht;
(2) Interpolieren jeder Zeitreihe auf eine festgelegte Abtastrate "fs";
(3) Auswählen einer der zwei PPG-Signalspuren aus den Aufzeichnungen der jeweiligen tragbaren Vorrichtungen als "Master" und Verwenden der Uhr der tragbaren Vorrichtung, die der ausgewählten Master-PPG-Signalspur entspricht, als eine Master-Uhr, die von da an als Referenz für die absolute Zeit verwendet wird; und
(4) Synchronisieren des Versatzes und der Uhrabweichung zwischen den zwei interpolierten Zeitreihensignalen.

2. Verfahren nach Anspruch 1, wobei Synchronisation des Versatzes und der Uhrabweichung umfasst:
(a) Ermitteln einer Verzögerung, ob positiv oder negativ, des "Slave"-Signals, die eine Korrelation zwischen den zwei interpolierten Zeitreihensignalen maximiert, wobei Ermitteln der Verzögerung Ermitteln einer Verzögerung umfasst, die eine maximale Kreuzkorrelation zwischen den zwei interpolierten Zeitreihensignalen ergibt;
(b) Anwenden eines Multiplikationsfaktors auf die festgelegte Abtastrate "fs", um etwaige Abweichungen auszugleichen, die die Slave-Uhr aufweisen kann; und
(c) Wiederholen der Schritte (a) und (b) bei der Suche des Multiplikationsfaktors innerhalb eines bestimmten Zeitintervalls, um einen optimalen Multiplikationsfaktor und eine Verzögerung zu ermitteln, die insgesamt eine höchste Kreuzkorrelation zwischen den zwei interpolierten Zeitreihensignalen ergeben.

3. Verfahren nach Anspruch 2, weiter umfassend:
Anwenden des optimalen Multiplikationsfaktors, in Bezug auf die Uhrabweichung, und Verzögerung, in Bezug auf Versatz auf das Beschleunigungssignal der Slave-Vorrichtung, um zwei genau synchronisierte Beschleunigungsaufzeichnungen zu erhalten, die dadurch verwendet werden können, die jeweiligen Aufzeichnungen für den speziellen Verwendungsfall zu verarbeiten, um die Messung des charakteristischen Verhaltens zu erhalten und/oder gegebene Verwendungsfallereignisse zu erkennen.

4. Verfahren nach Anspruch 1, wobei das Verfahren vor Synchronisierung weiter Kopplung der ersten tragbaren Vorrichtung und der zweiten tragbaren Vorrichtung, über separate Kopplungsvorgänge, mit der auf der Hub-Vorrichtung ausgeführten Hub-ausführbaren App umfasst, wobei die Hub-Vorrichtung mindestens eines von einem Smartphone, einer dedizierten Hub-Vorrichtung und einer Vorrichtung mit direkter Cloud-Konnektivität umfasst.

5. Verfahren nach Anspruch 1, wobei die tragbaren Vorrichtungen Bluetooth^{™}-fähige tragbare Vorrichtungen umfassen, die konfiguriert sind, sich mit der auf der Hub-Vorrichtung ausgeführten Hub-ausführbaren App zu synchronisieren, jedoch nicht miteinander.

6. Verfahren nach Anspruch 1, wobei jede tragbare Vorrichtung mit Mitteln für gekoppelte drahtlose Kommunikation mit der auf der Hub-Vorrichtung ausgeführten Hub-ausführbaren App ausgestattet ist, jedoch nicht miteinander.

7. Verfahren nach Anspruch 6, wobei die Mittel für gekoppelte drahtlose Kommunikation ein Bluetooth^{™}-gekoppeltes drahtloses Kommunikationsmodul der jeweiligen tragbaren Vorrichtung umfassen und wobei die Hub-Vorrichtung mindestens eines von einem Smartphone, einer dedizierten Hub-Vorrichtung und einer Vorrichtung mit direkter Cloud-Konnektivität umfasst.

8. Verfahren nach Anspruch 1, wobei Synchronisieren Übertragen jeweiliger Aufzeichnungen jedes der ersten und zweiten tragbaren Vorrichtung von jeder jeweiligen tragbaren Vorrichtung zu der auf der Hub-Vorrichtung ausgeführten Hub-ausführbaren App umfasst.

9. Hub-Vorrichtung, die konfiguriert ist, eine Hub-ausführbare App gemäß dem Verfahren nach Anspruch 1 auszuführen, um Aufzeichnungen von mindestens zwei tragbaren Vorrichtungen zu synchronisieren, die von einem Benutzer in einem speziellen Verwendungsfall getragen werden, wobei der spezielle Verwendungsfall einbezieht, dass die Hub-ausführbare App auf der Hub-Vorrichtung ausgeführt wird und dazu ausgelegt ist, ein charakteristisches Verhalten des Benutzers zu messen, oder dazu ausgelegt ist, eine Ersatzmessung einer vorübergehenden physikalischen Eigenschaft über einen oder mehrere Zeiträume durchzuführen.

10. Nicht-transitorisches, computerlesbares Medium, das mit Anweisungen verkörpert ist, die von einem Prozessor ausführbar sind, um den Prozessor zu veranlassen, das Verfahren nach Anspruch 1 auszuführen, um Aufzeichnungen von mindestens zwei tragbaren Vorrichtungen zu synchronisieren, die vom Benutzer in dem speziellen Verwendungsfall getragen werden, der dazu ausgelegt ist, das charakteristische Verhalten des Benutzers zu messen, oder dazu ausgelegt ist, eine Ersatzmessung einer vorübergehenden physikalischen Eigenschaft über einen oder mehrere Zeiträume durchzuführen.

## Revendications

1. Procédé pour synchroniser des enregistrements d'au moins deux dispositifs, pouvant être portés sur soi, portés par un utilisateur dans un cas d'utilisation spécifique, dans lequel le cas d'utilisation spécifique implique que l'application exécutable par un concentrateur soit exécutée sur un dispositif concentrateur et conçue pour mesurer un comportement caractéristique de l'utilisateur ou conçue pour effectuer une mesure de substitution d'une caractéristique physique transitoire sur une ou plusieurs périodes de temps, le procédé comprenant :
l'acquisition de premiers enregistrements via un premier dispositif pouvant être porté sur soi, le premier dispositif pouvant être porté sur soi pouvant fonctionner via une première horloge interne au premier dispositif pouvant être porté sur soi ;
l'acquisition simultanée de seconds enregistrements via un second dispositif pouvant être porté sur soi, le second dispositif pouvant être porté sur soi pouvant fonctionner via une seconde horloge interne au second dispositif pouvant être porté sur soi, et dans lequel la première horloge est **caractérisée par** une première dérive et un premier décalage d'horloge qui peuvent être différents d'une seconde dérive et d'un second décalage d'horloge de la seconde horloge ;
la synchronisation du premier dispositif pouvant être porté sur soi et du second dispositif pouvant être porté sur soi, via des opérations de synchronisation distinctes, l'application exécutable par un concentrateur étant exécutée sur le dispositif concentrateur pour le cas d'utilisation spécifique ; et
la mise en œuvre d'une correction de temps d'horloge, spécifique au comportement caractéristique qui est mesuré ou à la mesure de substitution de la caractéristique physique transitoire, des enregistrements des deux dispositifs pouvant être portés sur soi via l'application exécutable par un concentrateur avant le traitement des enregistrements respectifs pour le cas d'utilisation spécifique pour obtenir une mesure du comportement caractéristique ou la mesure de substitution de la caractéristique physique transitoire, dans lequel la correction de temps d'horloge garantit que les enregistrements respectifs ont été corrigés dans le temps de telle sorte que la différence entre les horloges respectives, ou dérive d'horloge, des deux dispositifs pouvant être portés sur soi ne dépasse pas la durée seuil de l'événement le plus court spécifique au comportement caractéristique qui est mesuré ou à la mesure de substitution de la caractéristique physique transitoire, dans lequel les premiers enregistrements comprennent au moins des données d'accéléromètre et de photopléthysmographie (PPG) en fonction du temps selon la première horloge, et dans lequel les seconds enregistrements comprennent au moins des données d'accéléromètre et de photopléthysmographie (PPG) en fonction du temps selon la seconde horloge.
**caractérisé en ce que** la mise en œuvre de la correction de temps d'horloge comprend l'alignement des première et seconde horloges sur la base d'une série temporelle d'intervalles entre les battements dérivés de données de PPG détectées via un capteur de PPG de chaque dispositif pouvant être porté sur soi respectif,
dans lequel la correction de temps d'horloge basée sur la série temporelle d'intervalles entre les battements comprend en outre :
(1) pour chaque enregistrement, la détection et la représentation automatiques (a) d'emplacements temporels de battements cardiaques sur chaque trace de signal de PPG et (b) d'intervalles entre les battements sous forme de deux séries temporelles échantillonnées de manière non égale, chaque échantillon dans chaque série temporelle indiquant une paire qui comprend (temps, intervalle entre les battements), dans lequel le terme « temps » correspond à un temps d'horloge ou à un emplacement temporel de chaque battement cardiaque détecté, et le terme « intervalle entre les battements » correspond à une distance temporelle jusqu'à un battement cardiaque suivant ou un précédent battement cardiaque ;
(2) l'interpolation de chaque série temporelle à un taux d'échantillonnage fixe « fs » ;
(3) la sélection de l'une des deux traces de signal de PPG des enregistrements des dispositifs respectifs pouvant être portés sur soi comme « maître » et l'utilisation de l'horloge du dispositif pouvant être porté sur soi qui correspond à la trace de signal de PPG maître sélectionnée comme horloge maîtresse qui sera désormais utilisée comme référence pour le temps absolu ; et
(4) la synchronisation du décalage et de la dérive d'horloge entre les deux signaux de séries temporelles interpolées.

2. Procédé selon la revendication 1, dans lequel la synchronisation du décalage et de la dérive d'horloge comprend :
(a) la trouvaille d'un délai, qu'il soit positif ou négatif, du signal « esclave » qui maximise une corrélation entre les deux signaux de séries temporelles interpolées, dans lequel la trouvaille du délai comprend la trouvaille d'un délai qui donne une corrélation croisée maximale entre les deux signaux de séries temporelles interpolées ;
b) l'application d'un facteur de multiplication à la fréquence d'échantillonnage fixe « fs » pour compenser toute dérive éventuelle de l'horloge esclave ; et
(c) la répétition des étapes (a) et (b) lors de la recherche du facteur de multiplication, dans un intervalle de temps spécifié, pour trouver un facteur de multiplication et un délai optimaux qui donnent la corrélation croisée globale la plus élevée entre les deux signaux de séries temporelles interpolées.

3. Procédé selon la revendication 2, comprenant en outre :
l'application du facteur de multiplication optimal, se rapportant à la dérive d'horloge, et du délai, se rapportant au décalage, au signal d'accéléromètre du dispositif esclave, pour obtenir deux enregistrements d'accélération parfaitement synchronisés qui peuvent, de ce fait, être utilisés pour traiter les enregistrements respectifs pour le cas d'utilisation spécifique pour obtenir la mesure du comportement caractéristique et/ou détecter des événements de cas d'utilisation donnés.

4. Procédé selon la revendication 1, dans lequel avant la synchronisation, le procédé comprend en outre l'appairage du premier dispositif pouvant être porté sur soi et du second dispositif pouvant être porté sur soi, via des opérations d'appairage distinctes, avec l'application exécutable par un concentrateur exécutée sur le dispositif concentrateur, dans lequel le dispositif concentrateur comprend au moins un d'un téléphone intelligent, d'un dispositif concentrateur dédié et d'un dispositif de connectivité directe au nuage.

5. Procédé selon la revendication 1, dans lequel les dispositifs pouvant être portés sur soi comprennent des dispositifs pouvant être portés sur soi compatibles Bluetooth^{™} configurés pour être synchronisés avec l'application exécutable par un concentrateur exécutée sur le dispositif concentrateur, mais pas les uns avec les autres.

6. Procédé selon la revendication 1, dans lequel chaque dispositif pouvant être porté sur soi est équipé de moyens de communication sans fil appariée avec l'application exécutable par un concentrateur exécutée sur le dispositif concentrateur, mais pas les uns avec les autres.

7. Procédé selon la revendication 6, dans lequel les moyens de communication sans fil appariée comprennent un module de communication sans fil appariée Bluetooth^{™} du dispositif respectif pouvant être porté sur soi et dans lequel le dispositif concentrateur comprend au moins un d'un téléphone intelligent, d'un dispositif concentrateur dédié et d'un dispositif de connectivité directe au nuage.

8. Procédé selon la revendication 1, dans lequel la synchronisation comprend le transfert d'enregistrements respectifs de chacun des premier et second dispositifs pouvant être portés sur soi depuis chaque dispositif respectif pouvant être porté sur soi à l'application exécutable par un concentrateur qui est exécutée sur le dispositif concentrateur.

9. Dispositif concentrateur configuré pour exécuter une application exécutable par concentrateur conformément au procédé selon la revendication 1, pour la synchronisation des enregistrements d'au moins deux dispositifs pouvant être portés sur soi portés par un utilisateur dans un cas d'utilisation spécifique, dans lequel le cas d'utilisation spécifique implique que l'application exécutable par le concentrateur soit exécutée sur le dispositif concentrateur et conçue pour mesurer un comportement caractéristique de l'utilisateur ou conçue pour effectuer une mesure de substitution d'une caractéristique physique transitoire sur une ou plusieurs périodes de temps.

10. Support non transitoire lisible par ordinateur comportant des instructions exécutables par un processeur pour amener le processeur à exécuter le procédé selon la revendication 1, pour la synchronisation des enregistrements d'au moins deux dispositifs, pouvant être portés sur soi, portés par l'utilisateur dans le cas d'utilisation spécifique conçu pour mesurer le comportement caractéristique de l'utilisateur ou conçu pour effectuer une mesure de substitution d'une caractéristique physique transitoire sur une ou plusieurs périodes de temps.
